# EUROPEAN PATENT APPLICATION

(11) **EP 4 417 206 A1**
(43) Date of publication of application: **21.08.2024**
(21) Application number: 22879739.5
(22) Date of filing: 09.10.2022
(51) Int. Cl.: A61K 31/4965, A61P 9/10

(54) **USE OF TETRAMETHYLPYRAZINE NITRONE DERIVATIVE IN TREATMENT OF ISCHEMIC STROKE**

(30) Priority: 13.10.2021 CN 202111193840; 30.09.2022 CN 202211214408
(71) Applicant: Guangzhou Magpie Pharmaceuticals Co., Ltd., Guangzhou, Guangdong 510005 (CN)
(72) Inventor: LIU, Wei, Guangzhou, Guangdong 510005 (CN); GU, Jianbo, Guangzhou, Guangdong 510005 (CN); YIN, Gang, Guangzhou, Guangdong 510005 (CN); WANG, Yuqiang, Guangzhou, Guangdong 510005 (CN)
(74) Representative: Uexküll & Stolberg
(86) International application number: PCT/CN2022/000140
(87) International publication number: WO 2023/060785

(57) **Abstract**

The present invention provides the use of tetramethylpyrazine nitrone derivatives in the treatment of ischemic stroke, including administration of a therapeutically effective amount of tetramethylpyrazine nitrone derivatives or pharmaceutical compositions thereof. In clinical experiments, it was unexpectedly found that the combination of TBN+t-PA significantly improved the NIHSS scores of neurological function in patients on the 30th and 90th days. In addition, the combination of TBN+t-PA significantly improved the neurological function mRS score of patients, and the complete cure rate (mRS score of 0) was significantly higher than that of the t-PA group.

## Description

### FIELD OF THE INVENTION

The present invention relates generally to the field of medicine and, more particularly, to the use of tetramethylpyrazine nitrone derivatives in the treatment of ischemic stroke.

### BACKGROUND OF THE INVENTION

Stroke refers to a disease characterized by cerebral ischemia or hemorrhagic injury as the main clinical manifestation. There are two common types of stroke: ischemic stroke and hemorrhagic stroke. Ischemic stroke is more common than hemorrhagic stroke, accounting for 60% to 70% of the total number of stroke. Ischemic stroke is mainly caused by cerebral embolism and the formation of local thrombosis. Arterial stenosis and thromboembolism caused by atherosclerosis, or cerebral embolism caused by emboli from the heart flowing to the brain with blood flow, causing blood supply obstruction to the brain, and then causing cerebral ischemia and hypoxia leading to brain tissue necrosis, and ultimately causing a series of neurological deficits and disorders in patients.

Stroke has become the first cause of death and the leading cause of disability among adults in China. Stroke has the characteristics of sudden onset and severe illness, and delayed treatment and treatment after onset is the major cause of death and disability. This critical illness has strict time window regulations for treatment, and the length of time from onset to receiving treatment directly determines the success rate of treatment. Acute cerebral infarction lesions are composed of a combination of the ischemic central area and the surrounding ischemic penumbra. The brain tissue damage in the ischemic central area is irreversible, and there are a large number of neurons in the reversible state in the ischemic penumbra. Blockage of intracranial blood vessels can lead to ischemia and hypoxia of brain tissue at the site of embolism, which can cause electrochemical cascade like reactions in brain cells, causing occasional activation of damaged intracellular signaling pathways and irreversible damage to the disease. But if the occlusion of blood vessels can be achieved in a short period of time, allowing for the reconstruction of blood circulation in the ischemic penumbra, it will be beneficial for the survival and functional recovery of neural cells in the region.

For the treatment of ischemic stroke, mechanical thrombectomy and drug thrombolysis are mainly used in clinical practice to achieve recanalization of occluded blood vessels. The commonly used thrombolytic drugs in clinical practice include streptokinase (SK), urokinase (UK), anisoylated plasminogen streptokinase activator complex (APSAC), recombinant staphylokinase (SAK), tissue plasminogen activator (t-PA), reteplase, and ranotipase, etc. Although fibrinolysis drugs can better dissolve thrombus, they usually have a shorter treatment time window, for example, recombinant tissue plasminogen activator alteplase must be used within 6 hours after stroke onset. In addition, even after successful recanalization of occluded blood vessels, most stroke survivors still suffer from permanent neurological deficits (Carmeliet P, Jain RK. Molecular mechanisms and clinical applications of angiogenesis. Nature. 2011; 473(7347):298-307).

### SUMMARY OF THE INVENTION

The present invention provided the use of tetramethylpyrazine nitrone derivatives and pharmaceutical compositions thereof in the treatment of ischemic stroke.

The tetramethylpyrazine nitrone derivatives have a structure of formula (I): 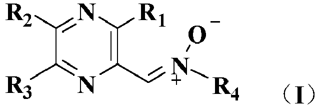 wherein R₁, R₂, and R₃ are independently C1-C6 alkyl; R₄ is sec-butyl, isobutyl, tert-butyl, cyclopentyl or cyclohexyl.

Preferably, the C1-C6 alkyl is methyl, ethyl, n-propyl, isopropyl, n-butyl, isobutyl, tert-butyl, or n-pentyl.

Further preferably, R₁, R₂, and R₃ are all methyl, and R₄ is tert-butyl, and therefore, the tetramethylpyrazine nitrone derivative is tetramethylpyrazine nitrone (TBN), i.e., cis-2-methyl-N-[(3,5,6-trimethylpyrazin-2-) methylene]2-propamine oxide, with a molecular formula of C₁₂H₁₉N₃O and structure below:

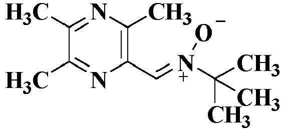

### Tetramethylpyrazine Nitrone (TBN)

The present invention provides the use of tetramethylpyrazine nitrone derivatives and pharmaceutical compositions thereof in the treatment of ischemic stroke, including administration of a therapeutically effective amount of tetramethylpyrazine nitrone derivatives or pharmaceutical compositions thereof, in combination with recanalization of occluded blood vessels.

The present invention also provides the use of the tetramethylpyrazine nitrone derivatives or pharmaceutical compositions thereof in the preparation of corresponding medicament.

According to an embodiment of the present invention, the recanalization of occluded blood vessels is mechanical thrombectomy or drug thrombolysis.

For drug thrombolysis the thrombolytic drug is selected from streptokinase (SK), urokinase (UK), anisoylated plasminogen streptokinase activator complex (APSAC), recombinant stapylokinase r-Sak, tissue type plasminogen activator ateplase (t-PA), prourokinase, nattokinase (NK), lumbrukinase, reteplase, tenecteplase (TNK-tPA), monteplase, lanoteplase (NPA), desmoteplase, pamipelase, solidase, tenecteplase (TNKase), ancrod, and snake venom thrombolytic enzyme, or a combination thereof.

According to an embodiment of the invention, the thrombolytic drug is tissue type plasminogen activator ateplase (t-PA).

According to an embodiment of the present invention, the tetramethylpyrazine nitrone derivatives are used in combination with mechanical thrombectomy.

The dosage of the thrombolytic drug of the present invention is determined by a clinical physician during thrombolysis on the basis of the severity and response of the disease, treatment-related toxicity, and the age, weight and health status of the patient. The method and frequency of administration can be determined according to a conventional method in the art.

The therapeutic effective amount of the tetramethylpyrazine nitrone derivatives of the present invention is 100-3000 mg/person/time. The specific dosage can be determined on the basis of the severity and response of the disease, treatment-related toxicity, and age, weight and health status of the patient. The specific method and frequency of administration can be determined according to a conventional method of the tetramethylpyrazine nitrone derivatives, for example, administered once or multiple times a day.

In some embodiments of the invention, the tetramethylpyrazine nitrone derivatives can be administered simultaneously or sequentially with the recanalization of occluded blood vessels to the patient. In some embodiments, the tetramethylpyrazine nitrone derivatives are administered simultaneously with the recanalization of occluded blood vessels. In some embodiments, the tetramethylpyrazine nitrone derivatives are administered after the recanalization of occluded blood vessels. In some embodiments, the tetramethylpyrazine nitrone derivatives are administered prior to the recanalization of occluded blood vessels. In some embodiments, the time difference between administration of the recanalization of occluded blood vessels and administration of the tetramethylpyrazine nitrone derivatives does not exceed 24 hours, while in some other embodiments, the time difference does not exceed 12 hours.

According to an embodiment of the invention, the ischemic stroke described herein is acute ischemic stroke.

According to an embodiment of the invention, a tetramethylpyrazine nitrone derivative as the active pharmaceutical ingredient is prepared with a pharmaceutically acceptable carrier into a pharmaceutical composition. The pharmaceutical composition can be in any form suitable for oral, sublingual, local inhalation (e.g., nasal spray), rectal, intramuscular, dermal, subcutaneous or intravenous administration. The amount of the pharmaceutical composition required for the treatment will vary depending on the route of administration, the nature of the treated condition, and the age, weight and health status of the patient, ultimately determined by the clinical physicians. The required dose can be provided as single or split doses (at appropriate intervals), such as two, three, or more times a day to achieve or meet the requirements of the treatment.

The term "pharmaceutically acceptable carrier" used herein refers to substances that do not interfere with the physiological effects of the tetramethylpyrazine nitrone derivatives and are non-toxic to mammals, including humans. The pharmaceutical composition of the tetramethylpyrazine nitrone derivatives can be formulated with the tetramethylpyrazine nitrone derivatives and pharmaceutically acceptable carriers via a method commonly known in the art. The pharmaceutical compositions can be prepared in a dosage form of, including but not limited to, solids, capsules, pills, suppositories, liquids (such as injections), oils, emulsions, sprays, gel, aerosols, inhalants, and patches.

The commonly used thrombolytic drugs in clinical practice, such as alteplase (t-PA), are used for thrombolytic treatment of ischemic stroke. After thrombolytic treatment, most stroke survivors still suffer from permanent neurological defects (Carmeliet P, Jain RK. Molecular mechanisms and clinical applications of angiogenesis. Nature. 2011;473(7347): 298-307).

In clinical trials, it was unexpectedly found that the combination of TBN+t-PA significantly improved the NIHSS score of neurological function in patients on the 14th and 90th days. In addition, the combination of TBN+t-PA significantly improved the neurological function mRS score of patients, and the complete cure rate (mRS score 0) was significantly higher than that of the t-PA alone; the prognosis (mRS score 0-2) of the combination of TBN+t-PA shown significantly improvement compared to the t-PA alone. The clinical experimental results indicated that the efficacy of the combination of TBN+t-PA after 90 days of the treatment is much better than that after 30 days, while there is no significant difference in efficacy between the 30 and 90 days of the treatment of t-PA alone. At the same time, TBN showed good safety and tolerability in clinical trials.

### BRIEF DESCRIPTION OF THE DRAWINGS

Figure 1 shows the percentage of patients with NHISS score of 0-1 in the t-PA group and the combination group;
Figure 2 shows the percentage of patients with mRS=0 in the t-PA group and the combination group;
Figure 3 shows the percentage of patients with mRS≤2 in the t-PA group and the combination group.

### DETAILED DESCRIPTION OF THE EMBODIMENTS

Some specific embodiments or examples of the present invention will be described below. It should be pointed out that the following embodiments or examples are only for further explanation of the present invention and cannot be understood as limiting the scope of the invention. Unless otherwise specified, all portions of the present invention are by weight, and all percentages are by mass.

The present invention adopts a multicenter, randomized, double-blind, parallel trial clinical research design. The patients with acute ischemic stroke included in the clinical trial center as subjects (all signed informed consent forms) were divided into non-thrombolytic group and thrombolytic group. The non-thrombolytic group consists of the saline group and the TBN group. The thrombolytic group consists of the t-PA group and the combination group of TBN + t-PA.

Subjects of the tests: Patients diagnosed with acute ischemic stroke were selected by clinical trial centers based on the diagnostic criteria of "Chinese Guidelines for the Diagnosis and Treatment of Acute Ischemic Stroke (2018)".

Administration method: TBN (1400mg) is dissolved in a sodium chloride injection bag (specification: 100mL) and administered intravenously for 30 minutes. Dose of t-PA: 0.9mg/Kg/dose; usage: added to physiological saline, 10% of the dose is administered via injected intravenous injection immediately within 1-2 minutes, and the remaining 90% is administered via intervenous drop infusion within 60 minutes. t-PA is administered only once on the first day of acute ischemic stroke. The medication schedule of TBN: D1-D7 administered twice a day, with an interval of 12 hours, for 7 consecutive days.

### Results:

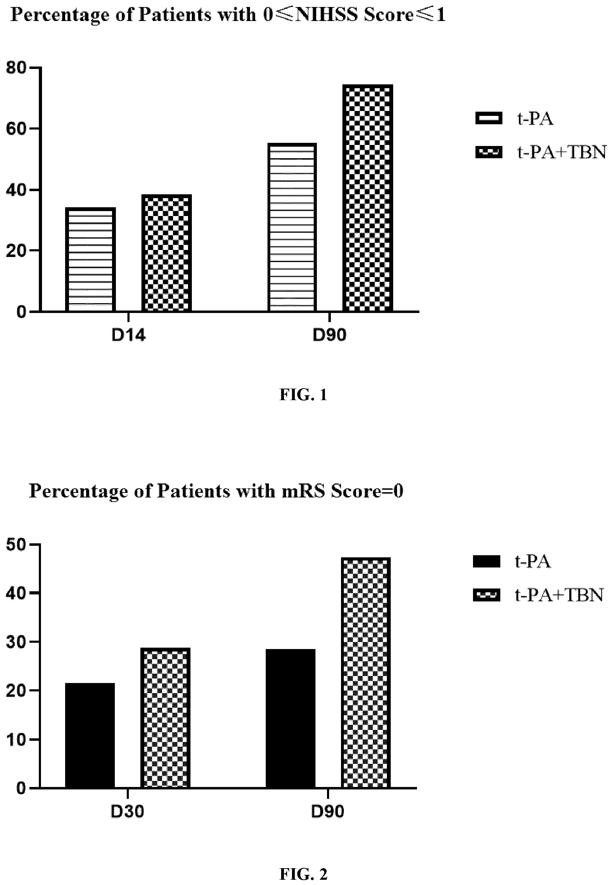

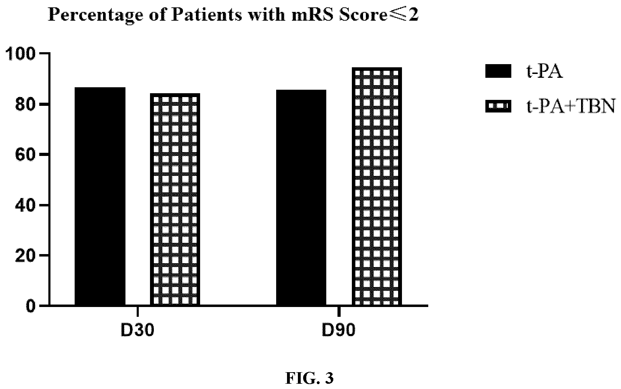

(1) In the non-thrombolytic group, compared to the saline group, the TBN group significantly improved the neurological function of patients on the 30th and 90th days, with a 0-1% increase in mRS compared to the saline group by 6.6 and 3.3 percentage points, respectively.
(2) In the thrombolytic group, compared to the t-PA group, the combination of TBN+t-PA significantly improved the patient's neurological function NIHSS scores on the 14th and 90th days. The specific results are shown in Figure 1, with NIHSS 0-1 scores increased by 12.57% and 34.54% (P=0.023), respectively, compared to the t-PA group. The combination of TBN+t-PA significantly improved the neurological function mRS score of patients on the 30th and 90th days, and the complete cure rate (mRS score 0) was shown in Figure 2, which was 33.80% and 65.73% (P=0.044), respectively, higher than the t-PA group; The results of a good functional prognosis (mRS score of 0-2) are shown in Figure 3, while, after 90 days, the combination group showed an increase of 10.50% compared to the t-PA group.
(3) After the completion of the experiment (on the 90th day), the Lsmeans mRS (1.12) of the combination group of TBN+t-PA improved by 0.49 points compared to the Lsmeans mRS (1.61) of the t-PA group, meaning an improvement of 1 point per 2 individuals. It can be understood that after treatment, compared to the t-PA group wherein 10 patients were unable to take care of themselves, in the combination therapy group only 5 patients were unable to take care of themselves, and 5 patients were able to take care of themselves, greatly improving the quality of life of these 5 patients.
(4) Regarding safety: tetramethylpyrazine nitrone derivatives shown good safety and tolerance.

It was found in the experiments that the treatment of ischemic stroke with TBN alone can improve the neurological function of patients, while the combination of TBN+t-PA has a better effect on improving the neurological function of patients. Compared with the t-PA group, the NIHSS scores of neurological function were significantly improved on the 30th and 90th day after administration of combination of TBN+t-PA. At the same time, research found that the efficacy of the TBN treatment on the 90th day was much better than that on the 30th day, while there was no significant difference in efficacy between the 30th and 90th days after the t-PA treatment.

The inventors have found that thrombolysis is more conducive to the arrival of the tetramethylpyrazine nitrone derivatives at the lesion site, and is more conducive to the survival of nerve cells and the recovery of neural function at the lesion site. Based on this discovery, in the treatment of ischemic stroke, mechanical thrombectomy or other thrombolytic drugs also result in more tetramethylpyrazine nitrone derivatives reaching the lesion site, which is also beneficial for the survival and functional recovery of nerve cells at the lesion site.

## Claims

1. Use of tetramethylpyrazine nitrone derivatives in the treatment of ischemic stroke, comprising administration of a therapeutically effective amount of tetramethylpyrazine nitrone derivatives or pharmaceutical compositions thereof, in combination with recanalization of occluded blood vessels; wherein, the tetramethylpyrazine nitrone derivatives have a structure of formula (I): 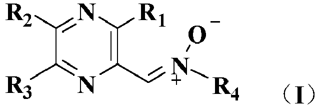 wherein R₁, R₂, and R₃ are independently C1-C6 alkyl; R₄ is sec-butyl, isobutyl, tert-butyl, cyclopentyl, or cyclohexyl.

2. The use according to claim 1, wherein the C1-C6 alkyl is methyl, ethyl, n-propyl, isopropyl, n-butyl, isobutyl, tert-butyl, or n-pentyl.

3. The use according to claim 1, wherein the tetramethylpyrazine nitrone derivatives have a structure of formula (TBN):

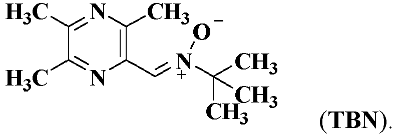

4. The use according to claim 1, wherein the recanalization of occluded blood vessels is mechanical thrombectomy or drug thrombolysis.

5. The use according to claim 4, wherein the recanalization of occluded blood vessels is drug thrombolysis, and thrombolytic drug for the drug thrombolysis is selected from streptokinase (SK), urokinase (UK), anisoylated plasminogen streptokinase activator complex (APSAC), recombinant stapylokinase r-Sak, tissue type plasminogen activator ateplase (t-PA), prourokinase, nattokinase (NK), lumbrukinase, reteplase, tenecteplase (TNK-tPA), monteplase, lanoteplase (NPA), desmoteplase, pamipelase, solidase, tenecteplase (TNKase), ancrod, and snake venom thrombolytic enzyme, or a combination thereof.

6. The use according to claim 5, wherein the thrombolytic drug is ateplase (t-PA).

7. The use according to claim 4, wherein the recanalization of occluded blood vessels is mechanical thrombectomy.

8. The use according to claim 1, wherein the ischemic stroke is acute ischemic stroke.

9. The use according to claim 1, wherein the therapeutically effective amount is 100-3000mg/person/time.

10. The use according to claim 1, wherein the pharmaceutical compositions comprise a therapeutically effective amount of tetramethylpyrazine nitrone or a pharmaceutically acceptable salt thereof, and a pharmaceutically acceptable carrier.

11. The use according to claim 8, wherein route of administration of the pharmaceutical compositions is oral, sublingual, local inhalation, rectal, intramuscular, dermal, subcutaneous or intravenous administration, or a combination thereof.

12. The use according to claim 8, wherein the pharmaceutical compositions are prepared in a dosage form with a pharmaceutically acceptable carrier, the dosage form being solids, capsules, pills, suppositories, liquids, oils, emulsions, sprays, gel, aerosols, inhalants, or patches.
